# EUROPÄISCHE PATENTSCHRIFT

(11) **EP 1 516 600 B1**
(45) Veröffentlichungstag und Bekanntmachung des Hinweises auf die Patenterteilung: **14.03.2007**
(21) Anmeldenummer: 04030668.0
(22) Anmeldetag: 18.09.2001
(51) Int. Cl.: A61F 2/06

(54) **Stent**
Stent
Stent

(43) Veröffentlichungstag der Anmeldung: 23.03.2005
(62) Teilanmeldung aus: 01122285.8
(73) Patentinhaber: Abbott Laboratories Vascular Enterprises Limited, Dublin 2 (IE)
(72) Erfinder: Calisse, Jorge, Dr., 10785 Berlin (DE)
(74) Vertreter: Schmitz, Hans-Werner

(56) Entgegenhaltungen:
- WO-A-99/17680
- WO-A-99/38458
- WO-A-99/49928
- US-B1- 6 261 318

## Beschreibung

Die Erfindung betrifft einen Stent gemäß dem Oberbegriff des Anspruches 1. Ein derartiger Stand ist aus der WO 99 4992 bekannt.

Aus dem Stand der Technik sind unterschiedlichste Ausgestaltungen von Stents, insbesondere Koronarstents als ballon- oder selbstexpandierbare Stents, vorbekannt. Der Stent bildet eine Gefäßprothese, die aus körperverträglichem Material besteht. Der Stent bzw. die Stentprothese wird dazu verwendet, Blutgefäße oder aber auch andere Körperöffnungen aufzuweiten und im aufgeweiteten Zustand zu halten. Zu diesem Zweck wird der Stent in einem nicht-expandierten Zustand, üblicherweise mit Hilfe eines Ballonkatheters, auf dem der Stent aufgecrimpt ist, im Körper des Patienten positioniert und nachfolgend expandiert. Beim Expandieren werden die einzelnen Stegbereiche des Stents verformt, so dass dieser dauerhaft in der expandierten Form bleibt.

Ein weiterer Stent der im Oberbegriff des Anspruches 1 angegebenen Art ist beispielsweise aus dem DE-U-297 08 689.8 bekannt. Bei diesem Stent sind als Verbinder zwischen den Stegmustern der Stegstruktur der Stentwand s-förmige Verbindungselemente vorgesehen. Obwohl diese Verbindungselemente im nicht-expandierten Zustand zu einer sehr flexiblen und im expandierten Zustand zu einer Stent-Konstruktion mit hoher Radialkraft bzw. Radialkraft-Aufnahmefähigkeit führen, ergeben sich jedoch Verbesserungsmöglichkeiten dahingehend dass nicht in jedem Falle ausgeschlossen werden kann, dass sich die Verbindungselemente beim Aufweiten aus der Wandebene nach aussen oder innen herauswölben und dadurch das umgebende Gewebe etwas in Mitleidenschaft gezogen wird bzw. die Bildung von Thrombosen oder Restenosen gefördert wird.

Es ist daher Aufgabe der vorliegenden Erfindung, einen Stent der im Oberbegriff des Anspruches 1 angegebenen Art zu schaffen, der im nicht-expandierten Zustand äusserst flexibel ist und bei dem beim Aufweiten verhindert werden kann, dass sich die Verbindungselemente aus der Wandfläche des Stents herausbewegen können.

Die Lösung dieser Aufgabe erfolgt durch die Merkmale des Anspruches 1.

Mit dem erfindungsgemäßen Stent wird eine sehr flexible Struktur beim Biegen und Komprimieren erreicht. Es ist insbesondere vorteilhafterweise möglich zu verhindern, dass beim Aufweiten des Stents die Verbindungselemente das Gewebe im expandierten Zustand des Stents beeinträchtigen, und dass das Lumen des Stentinneren vermindert wird.

Der Grund für diese vorteilhafte Wirkung ist vor allem darin zu sehen, dass sich die Verbindungselemente sowohl beim Biegen (wie zum Beispiel beim Platzieren des Stents in einem gekrümmten Gefäß) und beim Ausdehnen (wie zum Beispiel beim Aufweiten mittels des Ballons) verkürzen, was durch die besondere Anordnung der Verbindungsstege der Verbindungselemente mit den vorgesehenen Scharnieren bzw. Gelenken erreicht wird.

Die Unteransprüche haben vorteilhafte Weiterbildungen der Erfindung zum Inhalt.

Die Verbindungsstege der Verbindungselemente sind im nicht-expandierten oder komprimierten Zustand der Stegstruktur zumindest annähernd vorzugsweise exakt im rechten Winkel zueinander angeordnet.

Hierbei ist es möglich, die Breite der Verbindungsstege jeweils gleich zu machen oder, um eine erhöhte Flexibilität zu erreichen, die Breite des mittleren Verbindungssteges etwas geringer auszuführen als die Breite der an den mittleren Steg anschließenden Verbindungsstege.

Die Verbindungsstege, die mit den benachbarten Stegen der Stegstruktur verbunden sind, werden bei einer besonders bevorzugten Ausführungsform ebenfalls über Scharniere angebracht.

Als besonders bevorzugte und besonders einfache Ausführungsform für die Scharniere sind Filmscharniere vorgesehen.

Bevorzugterweise besteht der Stent aus einem biokompatiblen Material, das bei einer besonders bevorzugten Ausführungsform eine Nickel-Titan-Legierung oder Edelstahl darstellt.

Weitere Einzelheiten, Merkmale und Vorteile der Erfindung ergeben sich aus nachfolgender Beschreibung anhand der Zeichnung.

Es zeigt:
- Fig. 1: eine schematisch stark vereinfachte Prinzipdarstellung der Grundform des erfindungsgemäßen Stents,
- Fig. 2: eine Darstellung eines Teiles der Stegstruktur der Wand des Stents im nicht-expandierten Zustand,
- Fig. 3: eine Prinzipdarstellung der erfindungsgemäßen Verbindungselemente zur Erläuterung ihrer Funktionsweise, und
- Fig. 4A, B: jeweils eine der Fig. 2 entsprechende Darstellung der Anordnung des erfindungsgemäßen Stents in einem gekrümmten Gefäß.

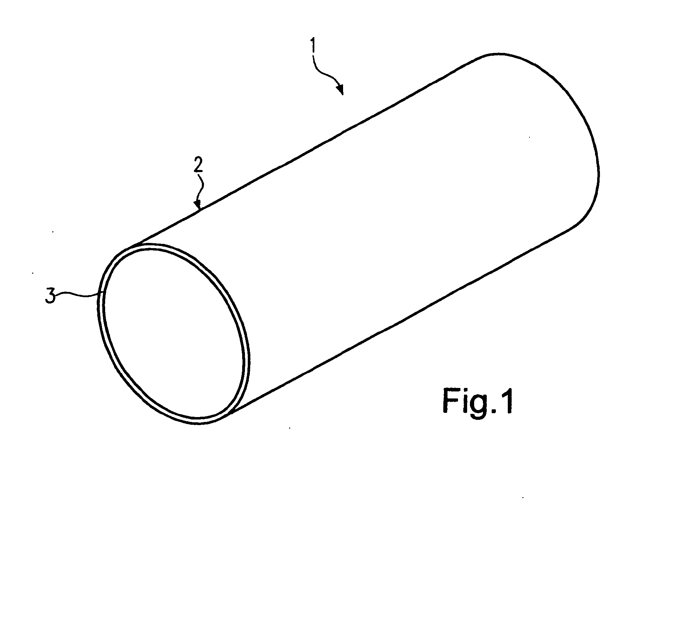

Fig. 1 zeigt den grundsätzlichen Aufbau eines erfindungsgemäßen Stents 1, der einen flexiblen, rohrförmigen Körper 2 mit einer Wand 3 aufweist, von der in Fig. 1 die Stirnansicht dargestellt ist. Der Stent gemäß der vorliegenden Erfindung kann als ballonexpandierbarer oder selbstexpandierbarer Stent ausgebildet sein.

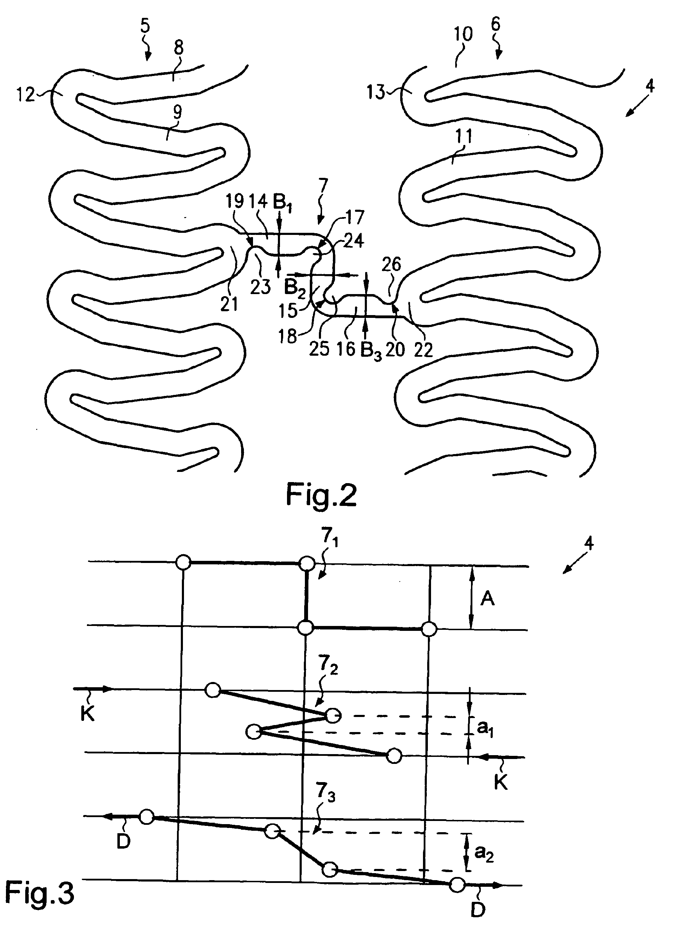

In Fig. 2 ist der Aufbau der Stegstruktur des erfindungsgemäßen Stents gezeigt.

Die Wand 3 des Körpers 2 des Stents 1 weist eine Stegstruktur 4 auf, die von einem nicht-expandierten Zustand in einen expandierten Zustand überführbar ist.

Hierbei weist die Stegstruktur 4 eine Vielzahl von benachbarten Stegmustern auf, von denen in Fig. 2 beispielhaft die Stegmuster 5 und 6 dargestellt sind. Die Stegmuster 5 und 6 sind untereinander durch zumindestens ein Verbindungselement 7 miteinander verbunden.

Zur Verdeutlichung des Aufbaus der Stegmuster 5 und 6 sind beispielhaft die Stege 8 und 9 sowie der diese Stege 8 und 9 verbindende Bogen 12 des Stegmusters 5 bzw. die entsprechenden Teile 10, 11 und 13 des Stegmusters 6 mit Bezugsziffern identifiziert.

Das Verbindungselement 7 weist drei im Winkel zueinander angeordnete Verbindungsstege 14, 15, 16 auf. Die Verbindungsstege 14 und 15 sind hierbei über ein Scharnier 17 und die Verbindungsstege 15 und 16 über ein Scharnier 18 miteinander verbunden. Der äussere Verbindungssteg 15 ist ferner über ein Scharnier 19 mit einem Bogen 21 und der zweite äussere Verbindungssteg 16 über ein Scharnier 20 mit dem Bogen 22 entsprechend der Stegstrukturen 5 bzw. 6 verbunden.

Bei der in Fig. 2 besonders bevorzugten Ausführungsform schließen die Verbindungsstege 14, 15 und 16 jeweils rechte Winkel zwischeneinander ein. Die Scharniere 17, 18, 19 und 20 sind als Filmscharniere ausgebildet, die durch das Vorsehen von annähernd halbkreisförmigen Ausmündungen 23, 24, 25 bzw. 26 in den jeweiligen Verbindungsstegen gebildet werden.

Ferner ist bei der in Fig. 2 dargestellten besonders bevorzugten Ausführungsform die Breite B1 gleich der Breite B2 und diese wiederum gleich der Breite B3 der Verbindungsstege 14, 15 bzw. 16. Zur Erhöhung der Flexibilität ist es jedoch möglich, insbesondere die Breite B2 des Verbindungssteges 15 im Vergleich zur Breite B1 und zur Breite B3 geringer auszubilden.

In Fig. 3 ist die Wirkungsweise des erfindungsgemäßen Verbindungselementes 7 innerhalb der Stegstruktur 4 verdeutlicht. Hierbei stellt die Anordnung 7₁ die Ausbildung des Verbindungselementes 7 gemäß Fig. 2 in schematisch vereinfachter Darstellung dar. Es ergibt sich ein Abstand A der der Länge des mittleren Verbindungssteges 15 entspricht.

Der Zustand 7₂ des Verbindungselementes 7 verdeutlicht die Stellung der Stege 14, 15 und 16 in einem komprimierten Zustand, der durch die beiden Pfeile K symbolisiert wird. Hierbei ergibt sich ein Abstand a1 zwischen den Scharnieren 17 und 18, der kleiner dem Abstand A ist.

Auch bei dem Zustand 7₃, der einem expandierten Zustand entspricht, ergibt sich ein Abstand a₂, der ebenfalls kleiner ist als der Abstand A.

Dies bedeutet, dass sich eine Verkürzung des Verbindungselementes 7 sowohl im komprimierten wie auch im expandierten Zustand ergibt, der es verhindert, dass die Verbindungselemente 7 aus der Wandebene der Stegstruktur 4 heraus vortreten, so dass insbesondere im implantierten Zustand eine Verletzung des umgebenden Gewebes des jeweiligen Volumens vermieden werden kann. Ferner ergibt sich der Vorteil, dass die Verbindungselemente in gekrümmten Gefässen eine gleichmäßige Wandabdeckung durch die Stegmuster und Flexibilität der gesamten Stentkonstruktion ergeben.

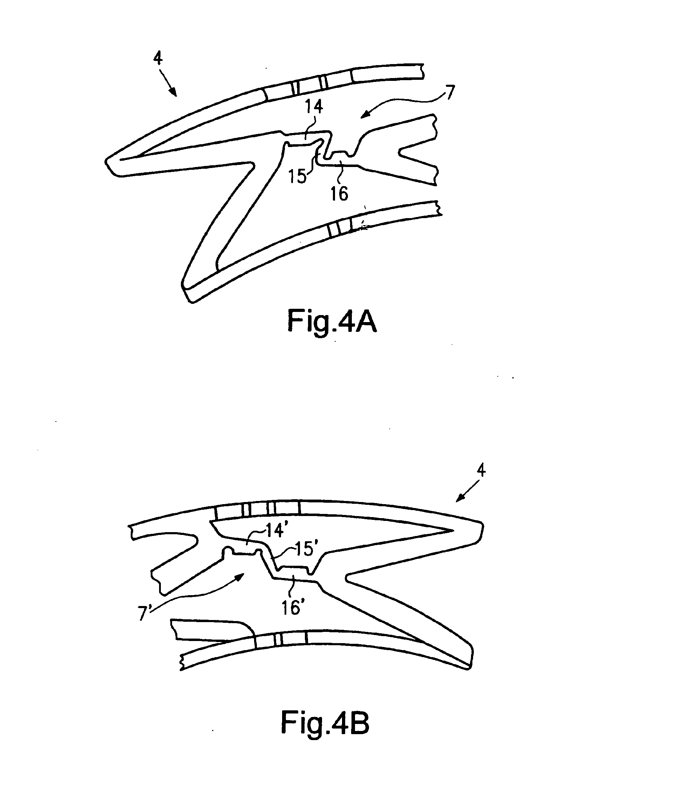

Hierzu ist auf die Figur 4A und 4B zu verweisen, in der ein Ausschnitt der Stegstruktur 4 jeweils bei einer Anordnung in einem gekrümmten Gefäß dargestellt ist.

Figur 4A zeigt hierbei den gestauchten Zustand des Verbindungselementes 7 mit seinen Stegen 14 bis 16, während 4B den gestreckten Zustand eines entsprechenden Verbindungselementes 7' mit seinen Stegen 14' bis 16' illustriert und verdeutlicht.

## Patentansprüche

1. Stent (1)
- mit einem rohrförmigen flexiblen Körper (2), dessen Wand (3) eine Stegstruktur (4) aufweist, die von einem nicht-expandierten Zustand in einen expandierten Zustand überführbar ist;
- wobei die Stegstruktur (4) eine Vielzahl von benachbarten Stegmustern (5,6) aufweist, die sich aneinander anreihende Stege (8,9 bzw. 10,11) aufweisen; und
- wobei zumindestens ein Paar der Stegmuster (5,6) durch zumindestens ein Verbindungselement (7) miteinander verbunden ist,
**dadurch gekennzeichnet,**
- **dass** das Verbindungselement (7) drei winklig zueinander angeordnete Verbindungsstege (14,15,16) mit zugeordneten Stegbreiten (B1,B2,B3) aufweist, die über Scharniere (17,18) miteinander verbunden sind, deren Breite geringer als zumindestens eine der Stegbreiten (B1,B2,B3) der Verbindungsstege (14,15,16) ist.

2. Stent nach Anspruch 1, **dadurch gekennzeichnet, dass** die Verbindungsstege (14-16) im nicht-expandierten oder komprimierten Zustand der Stegstruktur (4) zumindestens annähernd im rechten Winkel zueinander angeordnet sind.

3. Stent nach Anspruch 1 oder 2, **dadurch gekennzeichnet, dass** die Verbindungsstege (14-16) alle die gleiche Breite (B₁, B₂, B₃) aufweisen.

4. Stent nach Anspruch 1 oder 2, **dadurch gekennzeichnet, dass** die Verbindungsstege (14, 15, 16) unterschiedliche Breite aufweisen.

5. Stent nach Anspruch 1 oder 2, **dadurch gekennzeichnet, dass** die Breite (B₂) des mittleren Verbindungssteges (15) geringer als die Breite (B₁ bzw. B₃) der äusseren Verbindungsstege (14,16) ist.

6. Stent nach einem der Ansprüche 1 bis 5, **dadurch gekennzeichnet, dass** die äusseren Verbindungsstege (14,16) über je ein Scharnier (19,20) mit dem benachbarten Steg (21,22) der Wandstruktur (4) verbunden sind.

7. Stent nach einem der Ansprüche 1 bis 6, **dadurch gekennzeichnet, dass** die Scharniere (17-20) als Filmscharniere ausgebildet sind.

8. Stent nach einem der Ansprüche 1 bis 7, **dadurch gekennzeichnet, dass** das Material der Wand (3) des Körpers (2) aus körperverträglichem Material besteht.

9. Stent nach Anspruch 8, **dadurch gekennzeichnet, dass** die Stegstruktur (4) und die Verbindungselemente (7) aus einer Nickel-Titan-Legierung, aus Kunststoff oder nicht rostendem Stahl bestehen.

10. Stent nach einem der Ansprüche 1 bis 9, **dadurch gekennzeichnet, dass** die Länge der Verbindungsstege (14 - 16) gleich oder unterschiedlich ist.

## Claims

1. Stent (1)
- comprising a tubular flexible body (2), the wall (3) of which has a web structure (4) which can be transformed from a non-expanded state to an expanded state,
- the web structure (4) having a plurality of adjacent web patterns (5, 9) having webs (8, 9 and 10, 11 respectively) arranged side by side, and
- at least one pair of the web patterns (5, 6) being connected together by means of at least one connecting element (7),
**characterised in that**
- the connecting element (7) has three connecting webs (14, 15, 16) having associated web widths (B1, B2, B3) arranged at an angle relative to one another and connected together by means of hinges (17, 18) the width of which is smaller than at least one of the web widths (B1, B2, B3) of the connecting webs (14, 15, 16).

2. Stent according to claim 1, **characterised in that** the connecting webs (14-16) are arranged at least approximately at a right angle to one another in the non-expanded or compressed state of the web structure (4).

3. Stent according to claim 1 or claim 2, **characterised in that** the connecting webs (14-16) all have the same width (B₁, B₂, B₃).

4. Stent according to claim 1 or claim 2, **characterised in that** the connecting webs (14, 15, 16) have different widths.

5. Stent according to claim 1 or claim 2, **characterised in that** the width (B₂) of the central connecting web (15) is smaller than the width (B₁ or B₃) of the outer connecting webs (14, 16).

6. Stent according to one of claims 1 to 5, **characterised in that** the outer connecting webs (14, 16) are each connected to the adjacent web (21, 22) of the wall structure (4) by means of a hinge (19, 20).

7. Stent according to one of claims 1 to 6, **characterised in that** the hinges (17-20) are designed as film hinges.

8. Stent according to one of claims 1 to 7, **characterised in that** the material of the wall (3) of the body (2) consists of biocompatible material.

9. Stent according to claim 8, **characterised in that** the web structure (4) and the connecting elements (7) consist of a nickel-titanium alloy, plastic or stainless steel.

10. Stent according to one of claims 1 to 9, **characterised in that** the lengths of the connecting webs (14-16) are identical or different.

## Revendications

1. Endoprothèse vasculaire (1)
- avec un corps tubulaire flexible (2) dont la paroi (3) présente une structure nervurée (4) qui peut être amenée d'un état non dilaté à un état dilaté ;
- dans laquelle la structure nervurée (4) présente une pluralité de motifs de nervures voisins (5, 6) présentant des nervures (8, 9 ou 10, 11) disposées à la suite les unes des autres ; et
- dans laquelle au moins deux motifs de nervures (5, 6) sont reliés entre eux par un élément de liaison (7),
**caractérisée en ce que**
- l'élément de liaison (7) présente trois nervures de liaison (14, 15, 16), faisant un angle l'une par rapport à l'autre et ayant des largeurs de nervures (B₁, B₂, B₃) correspondantes, qui sont reliées entre elles par des charnières (17, 18) dont la largeur est inférieure à l'une au moins des largeurs de nervures (B₁, B₂, B₃) des nervures de liaison (14, 15, 16).

2. Endoprothèse vasculaire selon la revendication 1, **caractérisée en ce que**, dans l'état non dilaté ou comprimé de la structure nervurée (4), les nervures de liaison (14 à 16) sont disposées au moins approximativement à angle droit l'une par rapport à l'autre.

3. Endoprothèse vasculaire selon la revendication 1 ou la revendication 2, **caractérisée en ce que** les nervures de liaison (14 à 16) ont toutes la même largeur (B₁, B₂, B₃).

4. Endoprothèse vasculaire selon la revendication 1 ou la revendication 2, **caractérisée en ce que** les nervures de liaison (14, 15, 16) présentent des largeurs différentes.

5. Endoprothèse vasculaire selon la revendication 1 ou la revendication 2, **caractérisée en ce que** la largeur (B₂) de la nervure de liaison intermédiaire (15) est inférieure à la largeur (B₁ ou B₃) des nervures de liaison extérieures (14, 16).

6. Endoprothèse vasculaire selon l'une des revendications 1 à 5, **caractérisée en ce que** les nervures de liaison extérieures (14, 16) sont reliées chacune par une charnière (19, 20) à la nervure voisine (21, 22) de la structure de paroi (4).

7. Endoprothèse vasculaire selon l'une des revendications 1 à 6, **caractérisée en ce que** les charnières (17 à 20) sont réalisées sous la forme de charnières de type film.

8. Endoprothèse vasculaire selon l'une des revendications 1 à 7, **caractérisée en ce que** le matériau de la paroi (3) du corps (2) est un matériau biocompatible.

9. Endoprothèse vasculaire selon la revendication 8, **caractérisée en ce que** la structure nervurée (4) et les éléments de liaison (7) sont en alliage de nickel et de titane, en matière plastique ou en acier inoxydable.

10. Endoprothèse vasculaire selon l'une des revendications 1 à 9, **caractérisée en ce que** la longueur des nervures de liaison (14 à 16) est identique ou différente.
